(19) Europäisches Patentamt
European Patent Office
Office européen des brevets

(11) Numéro de publication: 0 027 075 A1

# (12) DEMANDE DE BREVET EUROPEEN

(21) Numéro de dépôt: 80401370.4

(22) Date de dépôt: 26.09.80

(51) Int. Cl.³: C 07 D 239/48, A 01 N 43/54

(30) Priorité: 09.10.79 FR 7925042

(43) Date de publication de la demande: 15.04.81 Bulletin 81/15

(84) Etats contractants désignés: BE DE FR GB IT LU NL

(71) Demandeur: P C U K PRODUITS CHIMIQUES UGINE KUHLMANN, Service Propriété Industrielle Tour Manhattan, F-92087 PARIS LA DEFENSE 2 Cédex 21 (FR)

(72) Inventeur: Balde, Daniel Henry, 13, rue Charles Friedel, F-75020 Paris (FR)
Inventeur: Boutemy, Gérard Emile Marcel, 19 Chemin des Résistants, Oncy-sur-Ecole F-91490 Milly la Foret (FR)

(54) **Alkylsulfinyl-5 pyrimidines, leur procédé de préparation et leurs utilisations comme herbicides.**

(57) Composés herbicides de formule:

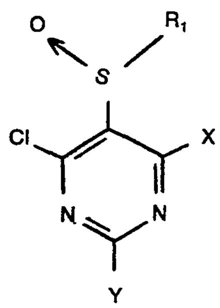

(I)

dans laquelle $R_1$ est un groupe alkyle ayant 1 à 5 atomes de carbone, X et Y sont identiques ou différents, chacun représentant un groupe amino $NH_2$ ou un groupe monoalkylamino ou dialkylamino dans lequel le ou les chaînes alkyle ont 1 à 5 atomes de carbone.

EP 0 027 075 A1

Alkylsulfinyl-5 pyrimidines, leur procédé de préparation et leurs utilisations comme herbicides

La présente invention a pour objet de nouveaux dérivés de la pyrimidine portant un groupe alkylsulfinyle en position 5, leur procédé de préparation et leurs utilisations en tant qu'herbicides.

Il est déjà connu, par exemple par les brevets français 2 031 422, 2 317 291, 2 137 933, 2 119 234, par la demande de brevet européen n° 78400062.2 publiée le 7 Février 1979 sous le numéro 681 et par l'article de MOSSINI, MAGGIALI, BRANCA Ateano Parmense, Acta Nat., 14 (1978), 119-126 des dérivés de la pyrimidine herbicides, mais ces dérivés ne portent jamais un groupe alkylsulfinyle.

Les nouvelles alkylsulfinyl-5 pyrimidines selon l'invention répondent à la formule générale :

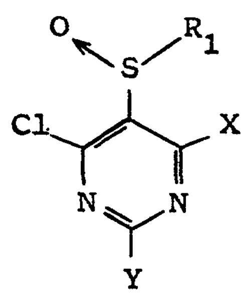

(I)

dans laquelle $R_1$ est un groupe alkyle ayant 1 à 5 atomes de carbone, en particulier un groupe méthyle, X et Y sont identiques ou différents, chacun représentant un groupe amino $NH_2$ ou un groupe monoalkylamino ou dialkylamino dans lequel le ou les chaînes alkyle ont 1 à 5 atomes de carbone.

Dans la formule (I) ci-dessus, de préférence au moins un des substituants X et Y est $NH_2$. Quand l'un des substituants X et Y est $NH_2$, c'est de préférence X qui est $NH_2$.

Les alkylsulfinyl-5 pyrimidines de formule (I) peuvent être préparées par oxydation, selon des méthodes connues en soi pour l'oxydation du groupe alkylthio en groupe alkylsulfinyle dans les composés organiques, des alkylthio-5 pyrimidines de formule :

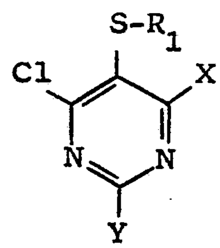

(II)

qui sont des composés connus.

Une telle réaction d'oxydation peut par exemple être réalisée au sein d'un solvant inerte, à une température comprise entre -10°C et 100°C, de préférence entre 0°C et 50°C, à l'aide d'oxydants tels que le dioxyde d'azote, le peroxyde d'hydrogène, l'ozone, les peroxydes organiques, en particulier les hydroperoxydes organiques et les acides peroxycarboxyliques. Les oxydants utilisés de préférence sont le peroxyde d'hydrogène et les acides peroxycarboxyliques. Les solvants inertes utilisés de préférence sont alors l'eau, les acides carboxyliques et les mélanges eau + acide carboxylique.

Les composés de formule (I) sont des herbicides qui ont la propriété de détruire, à faible dose et aussi bien dans les traitements de pré-levée que dans les traitements de post-levée, un grand nombre de plantes indésirables dans les cultures. En outre, aux doses auxquelles ils sont actifs vis-à-vis des plantes indésirables, les composés de formule (I) présentent fréquemment une phytotoxicité nulle ou très faible vis-à-vis des plantes cultivées, c'est-à-dire sont sélectifs des plantes cultivées, en particulier du blé, de l'orge, du maïs, du sorgho et du riz.

Les composés de formule (I) peuvent être employés aussi bien pour les traitements de pré-levée, auquel cas ils sont appliqués, avant émergence des plantes cultivées et des plantes indésirables, sur le sol où ont été effectués les semis de la plante cultivée, que pour les traitements de post-levée, auquel cas ils sont appliqués, après émergence des plantes cultivées et des plantes indésirables, sur les plantes elles-mêmes et le sol qui les entoure. La dose de composé de formule (I) appliquée est fonction du composé particulier choisi, de la nature des plantes (plante cultivée, plantes indésirables à détruire) et du mode de traitement (traitement de pré-levée ou traitement de post-levée). Cette dose peut aller de 200 g à 5 000g de matière active (composé de formule I) par hectare. Elle est de préférence de 500 g à 1 250 g de matière active par hectare.

Pour leur mise en oeuvre les composés herbicides de formule (I) sont de préférence incorporés dans des compositions qui contiennent, outre un ou plusieurs composés de formule (I), les additifs inertes habituellement utilisés en agriculture pour diluer les matières actives et pour faciliter leur conservation, leur mise en suspension aqueuse, leur adhérence sur le feuillage et leur résistance aux agents atmosphériques et aux dégradations biologiques. De tels additifs inertes sont bien connus de l'homme de l'art. Comme tels on peut citer des diluants solides (talc, silice, kieselguhr, craie, terre de diatomées, argile, etc...), des diluants liquides (eau, huiles minérales, solvants organiques tels que des cétones, des alcools, des hydrocarbures ou leurs dérivés chlorés), des agents tensio-actifs, etc... Les compositions selon l'invention peuvent être des suspensions aqueuses, des solutions dans un solvant organique émulsifiable dans l'eau, des poudres ou des granulés applicables en l'état ou dispersables en milieu gazeux. Ces compositions peuvent contenir 1 à 95 % en poids de matière active, la matière active étant constituée par un ou plusieurs composés

de formule (I).

Les exemples suivants illustrent l'invention sans la limiter. Les données relatives aux spectres de résonance magnétique nucléaire (spectres R.M.N.) des protons figurant dans ces exemples ont été obtenues en mettant les composés en solution dans le diméthylsulfoxyde deutéré et en utilisant comme référence le tétraméthylsilane.

EXEMPLE 1 : Amino-2 (ou -4) méthylamino-4 (ou -2) chloro-6 méthylsulfinyl-5 pyrimidine.

Dans un réacteur contenant 160 ml d'acide acétique et 14 g d'un mélange contenant 51 % en poids d'amino-2 chloro-6 méthylamino-4 méthylthio-5 pyrimidine et 49 % en poids d'amino-4 chloro-6 méthylamino-2 méthylthio-5 pyrimidine on ajoute, à la température de 20°C et en l'espace de 5 minutes, 3,7 g d'une solution aqueuse à 68,4 % en poids de peroxyde d'hydrogène. On laisse réagir pendant 48 heures à la température de 20°C à 25°C, puis on verse le contenu du réacteur sur un mélange d'eau et de glace. On neutralise par addition d'hydroxyde de sodium et recueille par filtration le précipité formé. On obtient ainsi 4 g d'un solide qui est un mélange d'amino-2 méthylamino-4 chloro-6 méthylsulfinyl-5 pyrimidine et d'amino-4 méthylamino-2 chloro-6 méthylsulfinyl-5 pyrimidine et qui fond à 191°C.

Spectre infra-rouge du produit obtenu :

bandes d'absorption de SO à 980 $cm^{-1}$ et 1 020 $cm^{-1}$

Spectre R.M.N. du produit obtenu :

protons de $O \leftarrow SCH_3$ : déplacement chimique $\delta = 2,87$ ppm

EXEMPLE 2 : Diamino-2,4 chloro-6 méthylsulfinyl-5 pyrimidine.

Dans un réacteur contenant 240 ml d'acide acétique et 19 g de diamino-2,4 chloro-6 méthylthio-5 pyrimidine on ajoute,

à la température de 20°C, 5,4 g d'une solution aqueuse à 69 % en poids de peroxyde d'hydrogène. Après deux heures de réaction tout est dissout. On laisse réagir pendant 90 heures encore, à la température de 20°C à 25°C, puis on élimine l'acide acétique par évaporation. Le résidu est lavé à l'eau et séché. On obtient ainsi 20,3 g de diamino-2,4 chloro-6 méthylsulfinyl-5 pyrimidine qui fond à 174°C-176°C.

Spectre infra-rouge du produit obtenu :

bande d'absorption de SO à 990 $cm^{-1}$

Spectre R.M.N. du produit obtenu :

protons de $O \leftarrow SCH_3$ : $\delta$ = 2,89 ppm

protons de $NH_2$ : $\delta$ = 7,25 ppm et 7,39 ppm

<u>EXEMPLE 3</u> : bis (éthylamino)-2,4 chloro-6 méthylsulfinyl-5 pyrimidine.

On opère comme à l'exemple 2 en partant de 50 g d'acide acétique, 5 g de bis (éthylamino)-2,4 chloro-6 méthylthio-5 pyrimidine et 1,1 g d'une solution aqueuse à 70 % en poids de peroxyde d'hydrogène. On obtient 3,3 g de bis (éthylamino)-2,4 chloro-6 méthylsulfinyl-5 pyrimidine qui fond à 135°C.

Spectre infra-rouge du produit obtenu :

bande d'absorption de SO à 1 020 $cm^{-1}$

Spectre R.M.N. du produit obtenu :

protons de $O \leftarrow SCH_3$ : $\delta$ = 2,84 ppm

<u>EXEMPLE 4</u> : Amino-4 éthylamino-2 chloro-6 méthylsulfinyl-5 pyrimidine.

On opère comme à l'exemple 2 en partant de 50 g d'acide acétique, 5 g d'amino-4 éthylamino-2 chloro-6 méthylthio-5 pyrimidine et 1,2 g d'une solution aqueuse à 70 % en poids de peroxyde d'hydrogène. On obtient 4,5 g d'amino-4 éthylamino-2 chloro-6 méthylsulfinyl-5 pyrimidine qui fond à 138°C.

Spectre infra-rouge du produit obtenu :

bande d'absorption de SO à 1 000 $cm^{-1}$

Spectre R.M.N. du produit obtenu :

protons de O⟵$SCH_3$ : $\delta$ = 2,83 ppm

EXEMPLE 5 : bis (isopropylamino)-2,4 chloro-6 méthylsulfinyl-5 pyrimidine.

On opère comme à l'exemple 2 en partant de 50 g d'acide acétique, 5 g de bis (isopropylamino)-2,4 chloro-6 méthylthio-5 pyrimidine et 1 g d'une solution aqueuse à 70 % en poids de peroxyde d'hydrogène. On obtient 4,5 g d'un produit visqueux constitué par la bis (isopropylamino)-2,4 chloro-6 méthylsulfinyl-5 pyrimidine.

Spectre infra-rouge du produit obtenu :

bande d'absorption de SO à 1 010 $cm^{-1}$

Spectre R.M.N. du produit obtenu :

protons de O⟵$SCH_3$ : $\delta$ = 2,85 ppm

EXEMPLE 6 : Ethylamino-2 (ou -4) isopropylamino-4 (ou -2) chloro-6 méthylsulfinyl-5 pyrimidine.

On opère comme à l'exemple 2 en partant de 50 g d'acide acétique, 5 g d'un mélange d'éthylamino-2 isopropylamino-4 chloro-6 méthylthio-5 pyrimidine et d'éthylamino-4 isopropylamino-2 chloro-6 méthylthio-5 pyrimidine et 1 g d'une solution aqueuse à 70 % en poids de peroxyde d'hydrogène. On obtient 4 g d'un produit visqueux qui est un mélange d'éthylamino-2 isopropylamino-4 chloro-6 méthylsulfinyl-5 pyrimidine et d'éthylamino-4 isopropylamino-2 chloro-6 méthylsulfinyl-5 pyrimidine.

Spectre infra-rouge du produit obtenu :

bande d'absorption de SO à 1 010 $cm^{-1}$

Spectre R.M.N. du produit obtenu :

protons de O⟵$SCH_3$ : $\delta$ = 2,85 ppm

EXEMPLE 7 : Amino-4 diéthylamino-2 chloro-6 méthylsulfinyl-5 pyrimidine.

On opère comme à l'exemple 2 en partant de 50 g d'acide acétique, 5 g d'amino-4 diéthylamino-2 chloro-6 méthylthio-5 pyrimidine et 1,1 g d'une solution aqueuse à 70 % en poids de peroxyde d'hydrogène. On obtient 2,5 g d'un produit visqueux constitué par l'amino-4 diéthylamino-2 chloro-6 méthylsulfinyl-5 pyrimidine.
Spectre infra-rouge du produit obtenu :
bande d'absorption de SO à 1 000 $cm^{-1}$
Spectre R.M.N. du produit obtenu :
protons de $O \leftarrow SCH_3$ : $\delta = 2,81$ ppm

EXEMPLE 8 : Amino-2 (ou -4) isopropylamino-4 (ou -2) chloro-6 méthylsulfinyl-5 pyrimidine.

On opère comme à l'exemple 2 en partant de 100 g d'acide acétique, 10,4 g d'un mélange des deux composés isomères amino-2 isopropylamino-4 chloro-6 méthylthio-5 pyrimidine (composé a) et amino-4 isopropylamino-2 chloro-6 méthylthio-5 pyrimidine (composé b) dans lequel le rapport $\frac{\text{composé a}}{\text{composé b}}$ est égal à 1,5, et 2,4 g d'une solution aqueuse à 70 % en poids de peroxyde d'hydrogène. On obtient 5 g d'un produit visqueux constitué par un mélange d'amino-2 isopropylamino-4 chloro-6 méthylsulfinyl-5 pyrimidine et d'amino-4 isopropylamino-2 chloro-6 méthylsulfinyl-5 pyrimidine.
Spectre R.M.N. du produit obtenu :
protons de $O \leftarrow SCH_3$ : $\delta = 2,85$ ppm

EXEMPLE 9

Dans cet exemple, les produits selon l'invention sont formulés sous forme de suspensions aqueuses contenant 5 ‰ d'un tensio-actif dénommé "TWEEN 20".

Les quantités de suspensions appliquées équivalent à 1 000 l/ha, et les dilutions réalisées sont calculées de façon à apporter les quantités de matière active suivantes :

$$D_1 = 2,5 \text{ kg/ha}$$
$$D_2 = 10 \text{ kg/ha}$$

Les suspensions sont appliquées par pulvérisation soit sur plantes âgées de 10 jours, ce qui permet d'étudier l'action de post-levée des produits, soit sur semences déposées à la surface du sol, ce qui permet d'étudier l'action de pré-levée. Ces semences sont recouvertes de 2 cm de terre juste après l'application.

Les plantes et graines sont disposées dans des conteneurs en plastique de 18x12x5 cm remplis d'une terre standard composée de 3 parties de sable, 1 partie de terreau et 1 partie d'argile. Après traitement, les conteneurs sont disposés sur une tablette à irrigation automatique dans une serre maintenue à 22°C et à un taux d'hygrométrie de 70 %.

Les plantes soumises aux essais sont le blé TRITICUM SP, le haricot PHASEOLUS SP, la betterave BETA SP, la moutarde SINAPIS SP, le pissenlit TARAXACUM SP et le maïs ZEA SP.

14 jours après le traitement pour les essais de post-levée et 21 jours après le traitement pour les essais de pré-levée, on compare l'énergie végétative des plantes dans les lots traités avec les produits selon l'invention à l'énergie végétative des plantes dans des lots non traités (lots témoins). Les résultats obtenus sont exprimés par un chiffre de 0 à 4 suivant l'échelle de notation suivante :

note 0 : aucun effet destructeur visible sur les plantes dans les lots traités

note 1 : le pourcentage de destruction des plantes dans les lots traités est de 1 % à 33 %

note 2 : le pourcentage de destruction des plantes dans les lots traités est de 34 % à 66 %

note 3 : le pourcentage de destruction des plantes dans les lots traités est de 67 % à 99 %

note 4 : le pourcentage de destruction des plantes dans les lots traités est 100 %

Les chiffres de 0 à 4 traduisent donc l'efficacité herbicide des produits vis-à-vis des plantes testées. Les résultats sont rassemblés dans le tableau I.

EXEMPLE 10

On conduit les essais comme à l'exemple 9. Seules changent les doses de matière active appliquées. Ces doses sont les suivantes :

$D_1$ = 0,625 kg/ha
$D_2$ = 1,25 kg/ha
$D_3$ = 2,5 kg/ha
$D_4$ = 5 kg/ha

Les résultats obtenus sont exprimés par un chiffre de 0 à 100 qui représente le pourcentage de destruction des plantes dans les lots traités et qui traduit donc l'efficacité herbicide des produits vis-à-vis des plantes testées. Le chiffre 0 indique que l'état des plantes est le même dans les lots traités et dans les lots témoins, ce qui correspond à une efficacité herbicide nulle, le chiffre 100 indique que les plantes sont entièrement détruites dans les lots traités, ce qui correspond à l'efficacité herbicide maximum.

Les résultats sont rassemblés dans les tableaux II et III.

EXEMPLE 11

Les produits selon l'invention sont formulés sous forme de suspensions aqueuses et sont appliqués en plein champ, par

pulvérisation, sur les plantes cultivées et les plantes adventices indésirables, l'application étant réalisée en post-levée des plantes, 15 jours après les semis des plantes cultivées. Les doses de matière active appliquées sont 1,25 ou 2,5 kg/ha.

Les plantes cultivées testées sont la tomate (Solanum lycopersicum), l'orge (Hordeum sp.), le soja (Glycine sp.), le tournesol (Helianthus sp.), le maïs (Zea sp.), la courgette (Cucurbita sp.), le blé (Triticum sp.), le haricot (Phaseolus sp.), la fève (Vicia fabae), le pois (Pisum sp.), l'avoine (Avena sp.), le sorgho (Sorghum sp.) et le riz (Oryza sp.). Les plantes adventices sont la moutarde (Sinapis sp.), le chenopode (Chenopodium sp.), l'amaranthe (Amaranthus sp.) le seneçon (Senecio sp.), la morelle (Solanum sp.), le ray-grass (Lolium sp.) et le panic (Panicum sp.).

Les résultats sont évalués 15 (J + 15) et 30 jours (J + 30) après l'application, l'évaluation étant effectuée de la même manière qu'à l'exemple 10. Les résultats sont rassemblés dans le tableau IV.

EXEMPLE 12

Les produits selon l'invention sont formulés sous forme de suspensions aqueuses et appliqués en plein champ, par pulvérisation, en pré-levée des plantes cultivées et des plantes adventices indésirables. L'application est effectuée sur le sol où ont été réalisés les semis des plantes cultivées, immédiatement après les semis. Les doses en matière active appliquées sont 1,25 ou 2,5 kg/ha.

Les plantes cultivées et les plantes adventices sont celles indiquées à l'exemple 11.

Les résultats sont évalués 30 jours (J + 30) et 60 jours (J + 60) après l'application, l'évaluation étant effectuée comme indiqué à l'exemple 10. Les résultats sont rassemblés dans le tableau V.

Tableau I

## EFFICACITE HERBICIDE

| Produits de l'exemple | POST-LEVEE | | | | | | | | | | | | PRE-LEVEE | | | | | | | | | | | |
|---|---|---|---|---|---|---|---|---|---|---|---|---|---|---|---|---|---|---|---|---|---|---|---|---|
| | $D_1$ = 2,5 kg/ha | | | | | | $D_2$ = 10 kg/ha | | | | | | $D_1$ = 2,5 kg/ha | | | | | | $D_2$ = 10 kg/ha | | | | | |
| | Bl | Ha | Be | Mo | Pi | Ma | Bl | Ha | Be | Mo | Pi | Ma | Bl | Ha | Be | Mo | Pi | Ma | Bl | Ha | Be | Mo | Pi | Ma |
| 1 | 0 | 3 | 4 | 4 | 4 | 0 | 1 | 3 | 4 | 4 | 4 | 1 | 0 | 2 | 4 | 4 | 4 | 0 | 1 | 4 | 4 | 4 | 4 | 0 |
| 2 | 0 | 0 | 2 | 4 | 4 | 0 | 0 | 2 | 4 | 4 | 4 | 0 | 0 | 0 | 4 | 4 | 4 | 0 | 0 | 0 | 4 | 4 | 4 | 0 |
| 3 | 0 | 4 | 4 | 4 | 4 | 0 | 0 | 4 | 4 | 4 | 4 | 0 | 0 | 2 | 4 | 2 | 4 | 0 | 0 | 4 | 4 | 4 | 4 | 2 |
| 4 | 1 | 3 | - | 4 | 4 | 0 | 1 | 4 | 4 | 4 | 4 | 2 | 1 | 4 | 4 | 4 | 4 | 1 | 3 | 4 | 4 | 4 | 4 | 2 |
| 5 | 0 | 1 | 4 | 4 | 4 | 0 | 2 | 3 | 4 | 4 | 4 | 1 | - | - | - | - | - | - | - | - | - | - | - | - |
| 6 | 1 | 2 | 4 | 4 | 4 | 0 | 2 | 4 | 4 | 4 | 4 | 0 | 0 | 0 | 1 | 0 | 0 | 0 | 0 | 0 | 4 | 1 | 4 | 0 |
| 7 | 2 | 4 | 4 | 4 | 4 | 0 | 4 | 4 | 4 | 4 | 4 | 0 | 1 | 3 | 4 | 4 | 4 | 0 | 3 | 3 | 4 | 4 | 4 | 0 |
| 8 | 3 | 4 | 4 | 4 | 4 | 0 | 4 | 4 | 4 | 4 | 4 | 1 | 2 | 3 | 4 | 4 | 4 | 0 | 3 | 4 | 4 | 4 | 4 | 0 |

Tableau II

APPLICATION DE POST-LEVEE

EFFICACITE HERBICIDE EN POURCENTAGE DE DESTRUCTION

| Produits de l'exemple | $D_1$ = 0,625 Kg/ha | | | | | | $D_2$ = 1,25 kg/ha | | | | | | $D_3$ = 2,5 kg/ha | | | | | | $D_4$ = 5 kg/ha | | | | | |
|---|---|---|---|---|---|---|---|---|---|---|---|---|---|---|---|---|---|---|---|---|---|---|---|---|
| | Bl | Ha | Be | Mo | Pi | Ma | Bl | Ha | Be | Mo | Pi | Ma | Bl | Ha | Be | Mo | Pi | Ma | Bl | Ha | Be | Mo | Pi | Ma |
| 1 | 0 | 40 | 100 | 80 | 100 | 0 | 0 | 60 | 100 | 90 | 100 | 0 | 0 | 80 | 100 | 100 | 100 | 0 | 0 | 100 | 100 | 100 | 100 | 0 |
| 2 | 0 | 55 | 55 | 71 | 100 | 0 | 0 | 64 | 92 | 100 | 100 | 0 | 0 | 83 | 93 | 100 | 100 | 0 | 0 | 95 | 100 | 100 | 100 | 0 |
| 4 | 7 | 64 | 100 | 100 | 100 | 0 | 15 | 70 | 100 | 100 | 100 | 0 | 36 | 80 | 100 | 100 | 100 | 0 | - | - | - | - | - | - |
| 7 | 19 | 80 | 100 | 100 | 100 | 0 | 30 | 80 | 100 | 100 | 100 | 0 | 75 | 93 | 100 | 100 | 100 | 0 | - | - | - | - | - | - |
| 8 | 6 | 73 | 80 | 93 | 73 | 0 | 23 | 74 | 100 | 100 | 100 | 0 | 48 | 91 | 100 | 100 | 100 | 0 | - | - | - | - | - | - |

Tableau III

## APPLICATION DE PRE-LEVEE

## EFFICACITE HERBICIDE EN POURCENTAGE DE DESTRUCTION

| Produits de l'exemple | $D_1$ = 0,625 kg/ha | | | | | | $D_2$ = 1,25 kg/ha | | | | | | $D_3$ = 2,5 kg/ha | | | | | | $D_4$ = 5 kg/ha | | | | | |
|---|---|---|---|---|---|---|---|---|---|---|---|---|---|---|---|---|---|---|---|---|---|---|---|---|
| | Bl | Ha | Be | Mo | Pi | Ma | Bl | Ha | Be | Mo | Pi | Ma | Bl | Ha | Be | Mo | Pi | Ma | Bl | Ha | Be | Mo | Pi | Ma |
| 1 | 0 | 60 | 0 | 0 | 100 | 0 | 0 | 61 | 82 | 49 | 100 | 0 | 10 | 65 | 100 | 100 | 100 | 0 | 15 | 90 | 100 | 100 | 100 | 0 |
| 2 | 0 | 0 | 88 | 79 | 88 | 0 | 0 | 0 | 99 | 100 | 98 | 0 | 0 | 0 | 100 | 100 | 100 | 0 | 0 | 0 | 100 | 100 | 100 | 0 |
| 4 | 0 | 0 | 30 | 20 | 90 | 0 | 0 | 0 | 50 | 70 | 90 | 0 | 30 | 100 | 100 | 100 | 100 | 0 | - | - | - | - | - | - |
| 7 | 0 | 0 | 40 | 18 | 9 | 0 | 0 | 3 | 50 | 35 | 58 | 0 | 8 | 90 | 98 | 80 | 83 | 0 | - | - | - | - | - | - |
| 8 | 0 | 70 | 68 | 40 | 4 | 0 | 0 | 78 | 78 | 70 | 78 | 0 | 50 | 83 | 95 | 80 | 100 | 5 | - | - | - | - | - | - |

Tableau IV EFFICACITE HERBICIDE EN POURCENTAGE DE DESTRUCTION

| Date de notation | J + 15 | | | | | | | | | J + 30 | | | | | | | | |
|---|---|---|---|---|---|---|---|---|---|---|---|---|---|---|---|---|---|---|
| Produit de l'exemple | 1 | | 2 | | 3 | | 4 | | 7 | 1 | | 2 | | 3 | | 4 | | 7 |
| dose en kg/ha | 1,25 | 2,5 | 1,25 | 2,5 | 1,25 | 2,5 | 1,25 | 2,5 | 1,25 | 1,25 | 2,5 | 1,25 | 2,5 | 1,25 | 2,5 | 1,25 | 2,5 | 1,25 |
| Plantes cultivées | | | | | | | | | | | | | | | | | | |
| Tomate | 60 | 65 | 20 | 30 | 10 | 30 | 70 | 85 | 10 | 60 | 70 | 40 | 50 | 10 | 30 | 80 | 95 | 30 |
| Orge | 20 | 30 | 0 | 15 | 10 | 10 | 15 | 80 | 30 | 10 | 25 | 20 | 30 | 15 | 20 | 10 | 80 | 40 |
| Soja | 50 | 80 | 0 | 40 | 0 | 50 | 70 | 80 | 30 | 45 | 90 | 0 | 30 | 10 | 15 | 95 | 100 | 35 |
| Tournesol | 30 | 40 | 0 | 10 | 20 | 30 | 50 | 90 | 25 | 40 | 55 | 0 | 15 | 35 | 70 | 90 | 100 | 0 |
| Maïs | 0 | 0 | 0 | 0 | 0 | 0 | 0 | 0 | 0 | 0 | 0 | 0 | 0 | 0 | 0 | 0 | 0 | 0 |
| Courgette | 30 | 60 | 0 | 50 | 20 | 25 | 30 | 100 | 0 | 50 | 80 | 30 | 50 | 0 | 20 | 20 | 100 | 0 |
| Blé | 0 | 30 | 10 | 30 | 5 | 10 | 40 | 90 | 10 | 0 | 10 | 25 | 40 | 0 | 0 | 10 | 80 | 0 |
| Haricot | 70 | 100 | 60 | 80 | 30 | 50 | 90 | 100 | 70 | 70 | 90 | 60 | 70 | 60 | 70 | 90 | 100 | 70 |
| Fève | 20 | 35 | 0 | 10 | 10 | 30 | 10 | 30 | 20 | 10 | 40 | 30 | 70 | 50 | 80 | 50 | 60 | 30 |
| Pois | 20 | 30 | 10 | 50 | 50 | 70 | 60 | 80 | 30 | 10 | 15 | 5 | 40 | 50 | 70 | 60 | 85 | 30 |
| Avoine | 0 | 30 | 25 | 50 | 40 | 50 | 30 | 90 | 10 | 0 | 50 | 20 | 40 | 20 | 30 | 20 | 80 | 0 |
| Sorgho | 0 | 10 | 0 | 0 | 0 | 15 | 0 | 5 | 0 | 0 | 10 | 0 | 0 | 0 | 10 | 0 | 5 | 0 |
| Riz | - | 20 | - | 20 | - | 15 | - | 20 | 10 | - | 10 | - | 15 | - | 5 | - | 15 | 5 |

Tableau IV (suite)

EFFICACITE HERBICIDE EN POURCENTAGE DE DESTRUCTION

| Date de notation | J + 15 | | | | | | | | | | J + 30 | | | | | | | | | |
|---|---|---|---|---|---|---|---|---|---|---|---|---|---|---|---|---|---|---|---|---|
| Produit de l'exemple | 1 | | 2 | | 3 | | 4 | | 7 | | 1 | | 2 | | 3 | | 4 | | 7 |
| Dose en kg/ha | 1,25 | 2,5 | 1,25 | 2,5 | 1,25 | 2,5 | 1,25 | 2,5 | 1,25 | | 1,25 | 2,5 | 1,25 | 2,5 | 1,25 | 2,5 | 1,25 | 2,5 | 1,25 |
| Plantes adventices | | | | | | | | | | | | | | | | | | | |
| Moutarde | 100 | 100 | 90 | 100 | 50 | 100 | 100 | 100 | 90 | | 100 | 100 | 80 | 100 | 60 | 100 | 100 | 100 | 80 |
| Chenopode | 100 | 100 | 100 | 100 | 100 | 100 | 100 | 100 | 100 | | 100 | 100 | 100 | 100 | 100 | 100 | 100 | 100 | 100 |
| Amaranthe | 80 | 100 | 75 | 100 | 65 | 100 | 100 | 100 | 100 | | 85 | 100 | 70 | 100 | 70 | 100 | 100 | 100 | 90 |
| Seneçon | 85 | 100 | 80 | 100 | 90 | 100 | 100 | 100 | 80 | | 75 | 100 | 80 | 100 | 85 | 100 | 100 | 100 | 100 |
| Morelle | 85 | 100 | 50 | 100 | 50 | 100 | 100 | 100 | 50 | | 85 | 100 | 60 | 100 | 70 | 100 | 100 | 100 | 60 |
| Ray grass | 60 | 100 | 30 | 50 | 40 | 95 | 80 | 100 | 35 | | 35 | 100 | 40 | 60 | 40 | 100 | 90 | 100 | 40 |
| Panic | 80 | 100 | 30 | 90 | 30 | 100 | 100 | 100 | 70 | | 80 | 100 | 20 | 100 | 40 | 100 | 100 | 100 | 75 |

Tableau V

EFFICACITE HERBICIDE EN POURCENTAGE DE DESTRUCTION

| Date de notation | J + 30 | | | | | | J + 60 | | | | | |
|---|---|---|---|---|---|---|---|---|---|---|---|---|
| Produit de l'exemple | 1 | | 2 | | 4 | 7 | 1 | | 2 | | 4 | 7 |
| Dose en kg/ha | 1,25 | 2,5 | 1,25 | 2,5 | 2,5 | 1,25 | 1,25 | 2,5 | 1,25 | 2,5 | 2,5 | 1,25 |
| Plantes cultivées | | | | | | | | | | | | |
| Tomate | - | - | 40 | 40 | 100 | 20 | - | - | 50 | 50 | 100 | 10 |
| Orge | 0 | 10 | 10 | 20 | 30 | 0 | 0 | 0 | 10 | 10 | 30 | 0 |
| Soja | 100 | 100 | 10 | 30 | 30 | 20 | 100 | 100 | 10 | 30 | 40 | 10 |
| Tournesol | 10 | 80 | 0 | 0 | 70 | 10 | 0 | 100 | 0 | 0 | 30 | 0 |
| Maïs | 0 | 0 | 0 | 0 | 0 | 0 | 0 | 0 | 0 | 0 | 0 | 0 |
| Courgette | 0 | 80 | 10 | 60 | 50 | 10 | 0 | 100 | 0 | 30 | 30 | 0 |
| Blé | 0 | 10 | 10 | 20 | 30 | 10 | 0 | 0 | 0 | 20 | 20 | 0 |
| Haricot | 50 | 60 | 50 | 70 | 100 | 20 | 40 | 50 | 40 | 70 | 100 | 20 |
| Fève | 0 | 0 | 0 | 40 | 100 | 30 | 0 | 0 | 0 | 0 | 30 | 30 |
| Pois | 0 | 0 | 0 | 50 | 100 | 10 | 0 | 0 | 0 | 10 | 40 | 10 |
| Avoine | 0 | 30 | 10 | - | - | 10 | 0 | 10 | 0 | 10 | - | 10 |
| Sorgho | 0 | 0 | 0 | 0 | 10 | 0 | 0 | 0 | 0 | 0 | 0 | 0 |
| Riz | 0 | 0 | 0 | 0 | 0 | 0 | 0 | 0 | 0 | 0 | 0 | 0 |

Tableau V (suite)

EFFICACITE HERBICIDE EN POURCENTAGE DE DESTRUCTION

| Date de notation | J + 30 | | | | | | J + 60 | | | | | |
|---|---|---|---|---|---|---|---|---|---|---|---|---|
| Produit de l'exemple | 1 | | 2 | | 4 | 7 | 1 | | 2 | | 4 | 7 |
| Dose en kg/ha | 1,25 | 2,5 | 1,25 | 2,5 | 2,5 | 1,25 | 1,25 | 2,5 | 1,25 | 2,5 | 2,5 | 1,25 |
| Plantes adventices | | | | | | | | | | | | |
| Moutarde | 80 | 100 | 50 | 100 | 100 | 60 | 70 | 100 | 35 | 100 | 100 | 60 |
| Chenopode | 60 | 90 | 60 | 95 | 100 | 70 | 60 | 100 | 50 | 90 | 100 | 90 |
| Amaranthe | 70 | 100 | 60 | 100 | 100 | 90 | 65 | 100 | 60 | 100 | 100 | 90 |
| Seneçon | 80 | 100 | 80 | 100 | 100 | 80 | 80 | 100 | 80 | 100 | 100 | 80 |
| Morelle | 75 | 100 | 70 | 100 | 100 | 85 | 70 | 100 | 65 | 100 | 100 | 75 |
| Ray grass | 60 | 90 | 30 | 50 | 100 | 30 | 60 | 80 | 20 | 45 | 100 | 40 |
| Panic | 50 | 100 | 20 | 60 | 100 | 60 | 40 | 100 | 10 | 50 | 100 | 50 |

Revendications de brevet

1. Composés de formule :

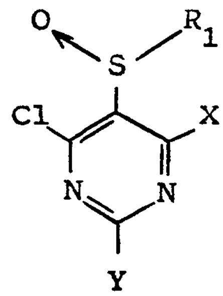

(I)

dans laquelle $R_1$ est un groupe alkyle ayant 1 à 5 atomes de carbone, X et Y sont identiques ou différents, chacun représentant un groupe amino $NH_2$ ou un groupe monoalkylamino ou dialkylamino dans lequel le ou les chaînes alkyle ont 1 à 5 atomes de carbone.

2. Composés selon la revendication 1 dans lesquels $R_1$ est un groupe méthyle.

3. Composés selon l'une quelconque des revendications 1 et 2 dans lesquels au moins un des substituants X et Y est $NH_2$.

4. Composés selon la revendication 3 dans lesquels X est $NH_2$ et Y est autre que $NH_2$.

5. Procédé de préparation des composés selon la revendication 1 caractérisé en ce qu'il consiste à oxyder les composés de formule :

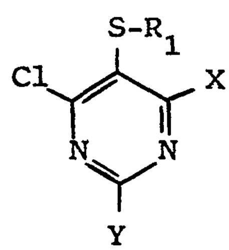

dans laquelle $R_1$, X et Y ont les mêmes significations que dans la formule (I) de la revendication 1.

6. Application en tant qu'herbicides des composés selon l'une quelconque des revendications 1 à 4.

Office européen des brevets

# RAPPORT DE RECHERCHE EUROPEENNE

0027075

Numéro de la demande

EP 80 40 1370

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| D | EP - A - 0 000 681 (PCUK)<br>* Pages 1,2,7-10 *<br>-- | 1-6 | C 07 D 239/48<br>A 01 N 43/54 |
| | CHEMICAL ABSTRACTS, vol. 90, no. 7, février 1979, page 614, réf. no. 54968y<br>Columbus, Ohio, US<br>& JP - A - 78 92789 (MITSUI TOATSU) (15-08-1978)<br>* En entier *<br>-- | 1-6 | DOMAINES TECHNIQUES RECHERCHES (Int. Cl. 3) |
| | CHEMICAL ABSTRACTS, vol. 91,(1979) page 714, réf. no. 57050z<br>Columbus, Ohio, US<br>& JP - A - 79 27580 (MITSUI TOATSU) (01-03-1979)<br>* En entier *<br>---- | 1-6 | C 07 D 239/48<br>A 01 N 43/54 |

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

☒ Le présent rapport de recherche a été établi pour toutes les revendications

| Lieu de la recherche | Date d'achèvement de la recherche | Examinateur |
|---|---|---|
| La Haye | 15-12-1980 | FRANCOIS |